# FASCICULE DE BREVET EUROPEEN

(11) **EP 3 645 127 B1**
(45) Date de publication et mention de la délivrance du brevet: **20.07.2022**
(21) Numéro de dépôt: 18748977.8
(22) Date de dépôt: 14.06.2018
(51) Int. Cl.: A61Q 19/08, A61K 8/92, A61K 8/9789, A61K 36/28, A61K 36/53

(54) **COMPOSITION COSMÉTIQUE COMPRENANT UNE HUILE ESSENTIELLE D'IMMORTELLE ET UN EXTRAIT D'ORIGANUM**
KOSMETISCHE ZUSAMMENSETZUNG MIT EINEM ÄTHERISCHEN ÖL AUS IMMORTELLE UND EINEM OREGANOEXTRAKT
COSMETIC COMPOSITION COMPRISING AN ESSENTIAL OIL OF IMMORTELLE AND AN EXTRACT OF ORIGANUM

(30) Priorité: 29.06.2017 FR 1756021
(43) Date de publication de la demande: 06.05.2020
(73) Titulaire: LABORATOIRES M & L, 04100 Manosque (FR)
(72) Inventeur: LEMAIRE, Géraldine, 04220 Corbières-en-Provence (FR); CENIZO, Valérie, 13650 Meyrargues (FR); KHAN, Cédric, 04860 Pierrevert (FR); PORTES, Pascal, 13540 Puyricard (FR)
(74) Mandataire: Renard, Emmanuelle
(86) Numéro de dépôt international: PCT/FR2018/051412
(87) Numéro de publication internationale: WO 2019/002714

(56) Documents cités:
- WO-A1-03/018730
- WO-A1-2017/077232
- WO-A2-2012/153064
- FR-A1- 2 999 926

## Description

### OBJET DE L'INVENTION

La présente invention concerne une composition cosmétique renfermant une huile essentielle d'immortelle et un extrait terpénique d'au moins une plante du genre *Origanum,* en particulier d'*Origanum majorana,* ainsi que son utilisation cosmétique pour le soin de la peau, en particulier pour lutter contre les signes cutanés du vieillissement et/ou le dessèchement de la peau.

### ARRIERE-PLAN DE L'INVENTION

Le vieillissement cutané est déterminé par des facteurs génétiques et environnementaux. On distingue habituellement le vieillissement intrinsèque, ou chronologique, qui affecte la peau comme les autres organes et correspond aux modifications inévitables liées à l'âge, du vieillissement extrinsèque qui est lié aux facteurs de l'environnement et en particulier à des modifications cliniques, histologiques et fonctionnelles caractéristiques de la peau liées à l'exposition solaire chronique et siégeant donc sur les zones photo-exposées.

Ces deux processus sont étroitement associés et dans les deux cas, la production d'espèces oxygénées réactives (ROS), conduisant à un stress oxydant est un élément déterminant du vieillissement de la peau. Il est défini comme un excès de radicaux libres présents dans l'organisme résultant d'une production excessive par divers mécanismes physiologiques ou de phénomènes toxiques exogènes (tels que le tabac, la pollution, la lumière bleue ou l'exposition aux UV). Ces espèces oxygénées réactives (ROS) et radicaux libres peuvent ainsi être générés soit par le métabolisme cellulaire, tel que la respiration mitochondriale, ou bien par la détoxification des xénobiotiques ou le rayonnement solaire.

En principe, cette production physiologique de radicaux libres est maîtrisée par les systèmes de défense cellulaires. Ceux-ci se trouvent toutefois affectés par différents facteurs tels qu'un déficit en anti-oxydants ou une baisse de l'activité des enzymes anti-oxydantes ou encore par la surproduction d'espèces oxygénées réactives, conduisant ainsi à un déséquilibre de la balance défenses anti-oxydantes / pro-oxydants et donc à un état de stress oxydant. Il est largement admis que ce stress oxydant joue un rôle important dans le vieillissement de la peau. Notamment, la dégradation des lipides et protéines cutanés sous l'effet du stress oxydant entraîne un relâchement cutané, la formation de rides et de taches pigmentaires et une perte d'élasticité de la peau et d'éclat du teint. Il a en outre été observé que le monoxyde d'azote produit en excès lors d'un déséquilibre de la balance redox inhibait la synthèse des protéines nécessaires à la cornification de l'épiderme et perturbait ainsi la fonction barrière.

D'autres phénomènes entraînent une perturbation de la fonction barrière, et notamment la réduction de la capacité de renouvellement et de différenciation des kératinocytes liée au vieillissement. L'altération de la barrière épidermique résulte en une déshydratation de l'épiderme et entraîne à son tour une plus grande perméabilité de la peau aux agents oxydants et polluants.

On comprend donc l'intérêt de disposer de moyens permettant de renforcer la barrière cutanée et de protéger la peau vis-à-vis du stress oxydant.

La Demanderesse a déjà proposé d'utiliser une huile essentielle d'immortelle, riche en composés terpéniques, comme agent anti-âge, en raison de ses propriétés anti-radicalaires et hydratantes (WO 03/018730), ou pour renforcer la fonction barrière (WO 2012/153064).

Parmi les autres plantes du bassin méditerranéen décrites comme efficaces contre le stress oxydant, on peut citer les polyphénols (EP 3 069 710) ou l'acide carnosique (US 9 040 066) issus de la marjolaine ou *Origanum majorana.* D'autres extraits de marjolaine sont également connus comme actifs anti-âge. Ainsi, la société BASF commercialise, sous la dénomination commerciale Dermagenist^{®}, un extrait aqueux de marjolaine riche en terpènes, pour ses propriétés inhibitrices de la méthylation de l'ADN. Il est ainsi suggéré que cet extrait pourrait être utile pour stimuler la formation des fibres élastiques qui se dégradent avec l'âge et sous l'effet de l'exposition aux UV (FR 2 999 926).

Il subsiste toutefois le besoin de proposer une composition cosmétique permettant de lutter efficacement contre les signes du vieillissement cutané et en particulier de renforcer la barrière cutanée et la réponse cutanée au stress oxydant.

### RESUME DE L'INVENTION

La Demanderesse a démontré que la combinaison d'un extrait terpénique *d'Origanum* avec une huile essentielle d'immortelle activait de manière synergique la voie Nrf2 qui est connue pour être liée à la réponse au stress oxydant. Le facteur de transcription Nrf2 est en effet un activateur de transcription puissant qui joue un rôle central dans l'expression inductible de nombreux gènes cytoprotecteurs en réponse aux stress oxydatif et électrophile. Les gènes cibles de Nrf2 sont en particulier impliqués dans la synthèse de glutathion, l'élimination des espèces réactives de l'oxygène et les enzymes de la détoxification cellulaire. La Demanderesse a également mis en évidence une augmentation, également synergique, de l'expression des gènes impliqués dans le processus de différenciation épidermique par la combinaison d'un extrait terpénique d'*Origanum* avec une huile essentielle d'immortelle. Il est ainsi apparu à la Demanderesse que la combinaison précitée permettait de formuler une composition cosmétique efficace contre les signes du vieillissement cutané et/ou le dessèchement de la peau.

Dans ce contexte, l'invention a pour objet une composition cosmétique comprenant une huile essentielle d'immortelle et un extrait terpénique d'au moins une plante du genre *Origanum.*

Elle a encore pour objet l'utilisation de la composition précitée pour lutter contre les signes du vieillissement cutané, notamment la formation de rides, le relâchement cutané et la perte d'éclat du teint, dus en particulier à l'exposition à la lumière bleue, aux UV, au tabac et/ou à la pollution et/ou pour lutter contre le dessèchement de la peau.

L'invention a également pour objet un procédé cosmétique pour lutter contre les signes du vieillissement cutané, notamment la formation de rides, le relâchement cutané et la perte d'éclat du teint, dus en particulier à l'exposition à la lumière bleue, aux UV, au tabac et/ou à la pollution, et/ou pour lutter contre le dessèchement de la peau, comprenant l'application topique sur la peau de la composition précitée.

### DESCRIPTION DETAILLEE

La présente invention met en œuvre une huile essentielle d'immortelle. Parmi les espèces d'immortelle susceptibles d'être utilisées selon l'invention, on citera toutes celles relevant du genre *Helichrysum* et en particulier *Helichrysum italicum* (ou *Helichrysum angustifolium* D.C), qui est l'immortelle d'Italie, *Helichrysum arenarium,* qui est l'immortelle des sables, et *Helichrysum stoechas* ou immortelle commune, sans que cette liste ne soit limitative. On utilise préférentiellement une huile essentielle d'*Helichrysum italicum,* quelle que soit la sous-espèce considérée.

Par "huile essentielle", on entend dans cette description le produit d'hydrodistillation ou d'entraînement à la vapeur des composés organiques volatils présents dans une partie quelconque de l'immortelle ou de la plante entière, et plus particulièrement dans ses parties aériennes comme par exemple ses fleurs ou sommités fleuries.

L'huile essentielle d'immortelle représente avantageusement de 0,001 à 5% en poids et préférentiellement de 0,001 à 0,1% en poids, par rapport au poids total de la composition selon l'invention.

Elle est associée, dans cette composition, à un extrait terpénique d'au moins une plante du genre *Origanum,* notamment des espèces *Origanum vulgare* et *Origanum majorana.* De préférence, la plante du genre *Origanum* appartient à l'espèce *Origanum majorana.*

On peut utiliser un extrait obtenu à partir de tout ou partie de la plante du genre *Origanum,* notamment la racine, la tige, l'écorce, la fleur, la graine, le germe et/ou la feuille et préférentiellement à partir des parties aériennes choisies parmi la tige, les feuilles et leurs mélanges, plus préférentiellement les feuilles.

L'extrait peut être obtenu par tout procédé convenant à l'extraction d'une fraction pondérale majoritaire de composés terpéniques et notamment par extraction à l'aide d'un solvant polaire protique comprenant, de préférence constitué par, au moins un solvant choisi parmi l'eau, un mono-alcool (tel que l'éthanol ou l'isopropanol), un glycol (tel que le propylène glycol ou le butylène glycol), un polyol (tel que le xylitol) et leurs mélanges. L'extraction peut être réalisée à froid (macération) ou à chaud, de préférence à une température de 4 à 20°C. La quantité de plante mise en œuvre peut représenter de 1 à 10% en poids, par rapport au poids de solvant(s). L'étape d'extraction est généralement suivie d'une étape de séparation des matières solides et du surnageant, par exemple par centrifugation, de récupération du surnageant, notamment par filtration, et éventuellement de distillation. L'extrait obtenu peut éventuellement être séché par atomisation et/ou lyophilisation. Des étapes de désodorisation et/ou de décoloration de l'extrait peuvent être réalisées après extraction, à un stade quelconque du procédé décrit précédemment.

Un tel extrait d'*Origanum majorana* est notamment disponible auprès de la société BASF sous la dénomination commerciale Dermagenist^{®}.

L'extrait d'*Origanum* représente avantageusement de 0,001 à 5% en poids et préférentiellement de 0,01 à 0,4% en poids, par rapport au poids total de la composition selon l'invention.

Comme il ressort des exemples ci-après, il a été démontré que la combinaison d'extraits de plantes selon l'invention permet d'activer de manière synergique l'expression des gènes impliqués dans la défense de la peau vis-à-vis des phénomènes d'oxydation et des gènes impliqués dans la régulation de la différenciation épidermique.

Par "synergie", on entend dans le cadre de cette description que l'effet obtenu en utilisant la combinaison d'extraits selon l'invention est significativement supérieur à l'effet obtenu en utilisant l'un quelconque des deux extraits.

La combinaison précitée est incluse dans une composition cosmétique qui renferme généralement un milieu physiologiquement acceptable, en particulier cosmétiquement acceptable, c'est-à-dire qui ne génère pas de picotements ou de rougeurs incompatibles avec une utilisation cosmétique. Ce milieu renferme de préférence une phase aqueuse (contenant par exemple l'extrait d'*Origanum*) et une phase grasse (contenant généralement l'huile essentielle d'immortelle). On préfère que la composition se présente sous forme d'émulsion huile-dans-eau ou eau-dans-huile ou d'une dispersion d'huile dans l'eau.

La phase aqueuse renferme de l'eau et éventuellement au moins un constituant choisi parmi les polyols et les gélifiants aqueux. L'eau représente avantageusement de 40 à 80%, par exemple de 60 à 70%, du poids total de la composition. Le polyol peut notamment être choisi parmi la glycérine, le propylène glycol, le butylène glycol, le pentylène glycol et leurs mélanges et il peut représenter de 5 à 30%, de préférence de 15 à 25%, du poids total de la composition.

Par "gélifiant aqueux", on désigne un composé polymérique capable d'immobiliser des molécules d'eau en s'hydratant et d'augmenter ainsi la viscosité de la phase aqueuse. Un tel gélifiant peut être choisi parmi : les polysaccharides, tels que : la cellulose et ses dérivés, les amidons modifiés, le carraghénane, l'agar agar, la gomme de xanthane et les gommes végétales telles que la gomme de guar ou de caroube ; les polymères synthétiques et notamment les homopolymères d'acrylate de sodium éventuellement réticulés, ainsi que les copolymères acryliques, en particulier les copolymères d'acrylate de sodium et/ou de (méth)acrylate d'alkyle et/ou de (méth)acrylate d'hydroxyalkyle et/ou de (méth)acrylate de (polyéthoxy)alkyle, avec éventuellement au moins un autre monomère, avantageusement l'acide 2-acrylamido-2-méthylpropane sulfonique (AMPS), ces copolymères étant éventuellement réticulés; et leurs mélanges.

De son côté, la phase grasse peut comprendre une ou plusieurs huiles volatiles et/ou non volatiles. Des exemples d'huiles volatiles sont les alcanes ramifiés, tels que l'isododécane, et les alcanes linéaires en C₁₀-C₁₃. Comme huiles non volatiles, on peut citer notamment :
- les esters d'acides et de mono-alcool choisis parmi : les mono- et polyesters d'acides linéaires saturés en C2-C10 (de préférence en C6-C10) et de mono-alcools linéaires saturés en C10-C18 (de préférence C10-C14), les mono- et polyesters d'acides linéaires saturés en C10-C20 et de mono-alcools ramifiés ou insaturés en C3-C20 (de préférence C3-C10) ; les mono- et polyesters d'acides ramifiés ou insaturés en C5-C20 et de mono-alcools ramifiés ou insaturés en C5-C20 ; les mono- et polyesters d'acides ramifiés ou insaturés en C5-C20 et de mono-alcools linéaires en C2-C4 ;
- les triglycérides d'acides gras en C6-C12, tels que les triglycérides d'acides caprylique et caprique et la triheptanoïne ;
- les acides gras ramifiés et/ou insaturés en C10-C20 (tels que les acides linoléique, laurique et myristique) ;
- les alcools gras ramifiés et/ou insaturés en C10-C20 (tels que l'octyldodécanol et l'alcool oléylique) ;
- les hydrocarbures tels que le squalane (C30), notamment le squalane végétal extrait de l'huile d'olive, et l'hémisqualane (C15) ;
- les carbonates de dialkyle, tels que le dicaprylyl carbonate et le diéthylhexyl carbonate ;
- les dialkyléthers tels que le dicaprylyl éther ; et
- leurs mélanges.

On peut également citer les huiles végétales qui contiennent un ou plusieurs des constituants précités.

Comme esters d'acides et de monoalcools, on peut notamment citer les monoesters tels que le mélange de caprate et caprylate de coco, le macadamiate d'éthyle, l'ester éthylique de beurre de karité, l'isostéarate d'isostéaryle, l'isononanoate d'isononyle, l'isononanoate d'éthylhexyle, le néopentanoate d'hexyle, le néopentanoate d'éthylhexyle, le néopentanoate d'isostéaryle, le néopentanoate d'isodécyle, le myristate d'isopropyle, le myristate d'octyldodécyle, le palmitate d'isopropyle, le palmitate d'éthylhexyle, le laurate d'hexyle, le laurate d'isoamyle, le nonanoate de cétostéaryle, le capylate de propylheptyle et leurs mélanges. D'autres esters utilisables sont les diesters d'acides et de monoalcools tels que l'adipate de disopropyle, l'adipate de diéthylhexyle, le sébaçate de diisopropyle et le sébaçate de diisoamyle.

Des exemples d'huiles végétales sont notamment les huiles de germe de blé, de tournesol, d'argan, d'hibiscus, de coriandre, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de cassis, d'onagre, de lavande, de bourrache, de millet, d'orge, de quinoa, de seigle, de carthame, de bancoulier, de passiflore, de rosier muscat, d'Echium, de cameline ou de camélia.

La phase grasse peut en outre comprendre au moins un structurant de phase grasse. Par "structurant de phase grasse", on entend un composé capable d'épaissir les huiles contenues dans la composition, choisi notamment parmi les cires, les gélifiants de phase grasse et les corps gras pâteux, ainsi que leurs mélanges.

Selon une forme d'exécution préférée, cette composition renferme en outre au moins un actif anti-âge, en particulier un actif adapté à prévenir et/ou traiter les rides, le relâchement cutané et/ou la formation de taches pigmentaires, qui peut notamment être choisi parmi les agents anti-radicalaires, les agents stimulant la différenciation et/ou la prolifération des kératinocytes et/ou des fibroblastes ; les agents stimulant la synthèse de glycosaminoglycanes et/ou de collagène et/ou de fibrilles d'ancrage dermoépidermique et/ou des fibres élastiques; les agents prévenant la dégradation du collagène et/ou des glycosaminoglycanes et/ou des fibrilles d'ancrage dermoépidermique et/ou des fibres élastiques ; les agents anti-glycation ; les agents dépigmentants et/ou inhibant la mélanogénèse ; et leurs mélanges.

Des exemples de tels actifs anti-âge sont notamment : l'acide ascorbique, ses sels, ses éthers et ses esters, notamment le glucoside d'ascorbyle ; l'adénosine ; le ribose ; les extraits de miel ; les protéines et glycoprotéines, extraites notamment d'amande douce ; les protéines végétales hydrolysées, notamment issues du riz, des graines d'hibiscus ou du lupin ; les polypeptides et les pseudodipeptides, tels que le chlorhydrate de carcinine, le palmitoyl pentapeptide-4 (Pal-Lys-Thr-Thr-Lys-Ser) et le palmitoyl tripeptide-38 commercialisés notamment par SEDERMA sous les dénominations commerciale Matrixyl^{®} 3000 et Matrixyl^{®} Synthe'6, respectivement, le palmitoyl tripeptide-8 commercialisé par la société LUCAS MEYER sous la référence commerciale Nutrazen^{®}, le pentapeptide-18 commercialisé par la société LIPOTEC sous la dénomination commerciale Leuphasyl^{®} Solution, le sh-decapeptide-9 commercialisé par la société SANDREAM sous la dénomination commerciale Neoendorphin^{®} et le palmitoyl hexapeptide-52 commercialisé par la société INFINITEC sous la référence commerciale X50 Myocept^{®} Powder ; les silanes tels que le mannuronate de méthylsilanol ; les arabinoxylanes, extraits en particulier de farine de seigle et les galactoarabinanes, issus notamment du mélèze ; l'acide hyaluronique et ses sels ; les polyphénols, extraits en particulier de mimosa ; les alphahydroxyacides, dont ceux extraits de citron; les extraits (généralement aqueux) de plantes telles que le trèfle d'eau, la pensée sauvage, la prêle des champs, la Mafane (*Acmella oleracea*), le chardon aux ânes (*Onopordum acanthium*), le millefeuille (*Achillea millefolium,* contenu notamment dans le produit Neurobiox^{®} de la société BASF), l'embelia (*Embelia concinna,* telle que commercialisée par la société SEPPIC), le figuier de Barbarie (*Opuntia ficus indica,* commercialisé notamment par MIBELLE AG BIOCHEMISTRY sous la dénomination commerciale AquaCacteen^{®}), la sauge (*Salvia officinalis,* vendue notamment par PROVITAL GROUP), *Vitex negundo* (commercialisé notamment par les LABORATOIRES EXPANSCIENCE sous la référence commerciale Neurovity^{®}), la châtaigne, la papaye, l'arganier, l'avoine, le tournesol, la pâquerette, la pivoine ou l'aneth ; les extraits aqueux d'algues et notamment de coralline, de janie rouge, d'*Ungaria pinnatifada,* d'*Alaria esculenta* ou de *Nannochlorosis oculata* ; les huiles essentielles, notamment de myrte ; les gluconates de zinc et/ou de cuivre ; et leurs mélanges.

En variante ou en plus, la composition utilisée selon l'invention peut comprendre au moins un agent tenseur. Il peut s'agir d'un polymère tenseur, capable de tendre la peau par action mécanique et de réduire ainsi l'apparence des rides et des ridules, en particulier d'un polysaccharide, notamment d'un extrait d'algue ou de plancton marin ou d'une gomme végétale. Il peut s'agir également d'un agent tenseur agissant par voie biologique du type « botox-like », par exemple , un extrait d'*Acmella;oleracea* commercialisé sous le nom de Gatuline^{®} Expression par la société GATTEFOSSE ; un extrait de graines d'hibiscus commercialisé sous le nom de Myoxinol^{®} LS9736 par la société BASF BCS ou encore un peptide de type Acétyl Hexapeptide-8 commercialisé sous le nom d'Argireline^{®} par la société Lipotec

La composition selon l'invention peut en outre contenir différents constituants qui peuvent être dispersés dans la phase grasse et/ou dans la phase aqueuse de l'émulsion, pour autant que ceux-ci soient compatibles avec une application topique sur la peau.

Elle peut ainsi renfermer au moins un émulsionnant huile-dans-eau ou eau-dans-huile, généralement non ionique, tel que des esters de polyoxyéthylène, des esters de sorbitane éventuellement polyéthoxylés, des esters d'acides gras et de glycérol éventuellement polyéthoxylés, des éthers d'alcools gras et de sucre tels que les alkyl glucosides, et leurs mélanges. Les émulsionnants peuvent représenter de 2 à 10% et de préférence de 4 à 6% du poids total de la composition. On préfère toutefois que la composition selon l'invention soit dépourvue d'émulsionnant.

La composition selon l'invention peut également comprendre une ou plusieurs charges pulvérulentes, qui se présentent avantageusement sous forme de microparticules poreuses ou creuses, de préférence poreuses. Ces microparticules sont en principe sensiblement sphériques. Ces charges peuvent notamment être choisies parmi :
- les charges organiques telles que : les poudres de polysaccharides et en particulier d'amidon natif, d'amidon modifié ou de cellulose ; les poudres de polymères acryliques tels que le poly(méthacrylate de méthyle), de polyamides ou de polyoléfines ; les poudres d'algues séchées telles que *Corallina officinalis ;*
- les charges inorganiques telles que la silice, les argiles, la perlite et le talc ;
- et leurs mélanges.

Comme charge inorganique, on préfère utiliser la silice.

Ces charges peuvent représenter de 1 à 5% en poids, par rapport au poids total de la composition.

La composition selon l'invention peut en outre comprendre des additifs choisis notamment parmi des agents photoprotecteurs organiques et/ou inorganiques, actifs dans la lumière bleue et/ou l'UVA et/ou l'UVB ; des polymères filmogènes à base de polysaccharides, capables de former un film protecteur anti-pollution, tels que les produits commercialisés par SOLABIA sous les dénominations commerciales Pollustop^{®} et Solashield^{®} ; des agents desquamants tels que des α- et β-hydroxyacides ; des particules exfoliantes ; des parfums ; des agents anti-oxydants ; des agents séquestrants ; des ajusteurs de pH ; des conservateurs ; des pigments ; des colorants ; et leurs mélanges.

Cette composition peut se présenter sous toute forme adaptée à une application topique sur la peau et notamment sous forme de lait, de crème, de lotion, de gel, de pâte ou de film. Il s'agit généralement d'une composition non rincée et en particulier d'une composition de soin, d'une composition de maquillage, telle qu'un fond de teint, ou d'une composition solaire.

La composition selon l'invention peut être appliquée sur au moins une zone du corps d'une personne présentant des signes de vieillissement cutané (par exemple d'au moins 30 ans, voire d'au moins 40 ans, et plus particulièrement sur le visage, le cou et/ou le décolleté. En variante, elle peut être appliquée sur l'ensemble du corps, notamment sur le buste, les bras, les jambes et le ventre en vue de lutter contre le dessèchement de la peau.

Cette composition peut être appliquée une ou plusieurs fois par jour, par exemple matin et/ou soir, sur les zones à traiter.

En variante, la composition selon l'invention peut être une composition rincée utilisée pour le soin de la peau, en particulier du visage et éventuellement du corps. Dans ce cas, elle peut par exemple être utilisée comme masque ou comme pâte de gommage.

### EXEMPLES

L'invention sera mieux comprise à la lumière des exemples suivants, qui sont donnés à titre purement illustratif et n'ont pas pour but de limiter la portée de l'invention, définie par les revendications annexées.

### Exemple 1 : Test ex-vivo sur explant de peau humaine

On a réalisé une analyse transcriptomique afin de mettre en évidence l'effet de la combinaison de plantes selon l'invention sur l'expression de certains gènes de la peau.

Le transcriptome est l'ensemble des ARN messagers issus de l'expression d'une partie du génome dans un tissu ou un type cellulaire donné. La caractérisation et la quantification du transcriptome permettent d'identifier les gènes régulés dans des conditions particulières, de déterminer les mécanismes de régulation de ces gènes et de définir les réseaux d'expression ou des voies d'activation de ces gènes. Une des techniques utilisées pour mesurer simultanément le niveau d'expression d'un grand nombre d'ARN messagers différents est celle de la puce à ADN. Les puces à ADN permettent de mesurer et de visualiser très rapidement les différences d'expression à l'échelle d'un génome complet.

### 1) Matériel et méthodes

### Préparation des échantillons

Un extrait aqueux de feuilles de marjolaine (*Origanum majorana*)*,* une huile essentielle d'immortelle (*Helichrysum italicum*)*,* obtenue par hydrodistillation, ou la combinaison des deux extraits, ont été mis en présence d'explants de peau en survie. L'huile essentielle d'immortelle a été solubilisée à 10% dans le diméthylsulfoxide (DMSO) avant d'être appliquée dans le milieu de culture. L'extrait de marjolaine a été directement dilué dans le milieu de culture. 4 explants de peau provenant de plastie abdominale d'une patiente de 34 ans ont été incubés indépendamment, en présence de l'extrait de Marjolaine, de l'huile essentielle d'immortelle ou de leur association pendant 24h dans un milieu « skin culture médium » de la société Biopredic à 37°C à 5% de CO₂ en atmosphère humide (98%) à l'interface air-liquide. 4 explants de peau non traités ou traités avec le solvant DMSO à 1% ont été incubés dans les mêmes conditions.

Le Tableau 1 ci-dessous récapitule les concentrations appliquées pour chaque composition.

**Tableau 1**

| **Composition** | **Pourcentage (%)** |
|---|---|
| Extrait de Marjolaine | 0,4 |
| Huile essentielle d'immortelle | 0,1 |
| Extrait de Marjolaine et huile essentielle d'immortelle | 0,4 |
| | 0,1 |

### Extraction des ARNs totaux

Après incubation, les explants de peau ont été broyés dans le tampon préconisé par le fabricant du kit d'extraction d'ARN RNeasy mini kit (Qiagen, Hilden, Allemagne) puis les ARN totaux ont été extraits en utilisant ce même kit. Les ARN totaux ont été quantifiés puis leur qualité a été vérifiée en utilisant l'appareil d'électrophorèse BIOanalyzer 2100 avec son kit d'analyse RNA 6000 Nano LabChip Kit (Agilent Technologies, Santa Clara, USA).

### Mesure de l'expression génique sur puce à oligonucléotides et acquisition des données :

Les puces à oligonucléotides de 60mers sur des lamelles en verre 1"x3", (SurePrint G3 Human Gene Expression 8x60K v2 Microarray - G4851B, Agilent Technologies, Santa Clara, USA) ont été utilisées pour l'analyse de l'expression des gènes.

Une documentation ainsi que le protocole expérimental complet est disponible sur le site d'Agilent Technologies.

En bref, la rétrotranscription et l'amplification des ARN totaux en ADNc puis en ARNc et leur marquage avec la cyanine 3 est réalisé à l'aide du kit Low Input Quick Amp Labeling kit (Agilent Technologies). La purification des ARNc a été effectuée à l'aide du kit RNeasy mini kit (Qiagen, Hilden, Allemagne) et l'hybridation avec le kit Microarray hybridization chamber kit (Agilent Technologies, Santa Clara, USA). Les puces ont été ensuite scannées en utilisant le scanner SureScan (Agilent Technologies, Santa Clara, USA) et le logiciel Scan control (Agilent Technologies, Santa Clara, USA). Les images scannées ont ensuite été extraites et normalisées avec le logiciel Feature Extraction (Agilent Technologies, Santa Clara, USA).

L'analyse statistique des données a été réalisée à l'aide du logiciel Genespring GX 13 (Agilent Technologies, Santa Clara, USA).

Le logiciel Ingenuity Pathways Analysis (Ingenuity^{®} Pathway Analysis (IPA), Ingenuity Systems, Redwood City, CA, USA - http://www.ingenuity.com) a été ensuite utilisé pour analyser et prédire l'état d'activation des voies biologiques modulées par l'extrait de Marjolaine, l'huile essentielle d'immortelle ou leur association.

Pour chaque condition de traitement, 4 expériences indépendantes de marquage ont été réalisées (soit 4 puces oligonucléotides par condition, correspondant aux 4 explants de peau traités indépendamment pour chaque condition) pour augmenter la reproductibilité des données.

Les gènes exprimés par les explants de peau traités ont été considérés comme étant induits ou inhibés si leur niveau d'expression différentielle était supérieur à un facteur 2 comparativement à ceux des explants contrôles. Les explants non traités ont servi de contrôle pour la condition traitée par l'extrait de Marjolaine et les explants traités avec le solvant DMSO à 1% ont servi de contrôle pour la condition traitée avec l'huile essentielle d'immortelle ou la combinaison de l'extrait de Marjolaine et l'huile essentielle d'immortelle. Un test de Student modéré corrigé du taux de faux positifs a été appliqué avec la procédure de Benjamini & Hochberg grâce au logiciel GeneSpring afin de calculer la différence statistique d'expression génique entre les explants non traités ou traités avec le solvant DMSO et les explants traités avec les actifs ou leur association. Les gènes dont la valeur p était inférieure ou égale à 0,05 ont été considérés comme différentiellement exprimés.

Ces gènes ont ensuite été analysés par le logiciel Ingenuity Pathways Analysis. Ce logiciel permet une analyse fonctionnelle des gènes régulés dans les différentes conditions de traitement, une analyse des voies de signalisation dans lesquelles s'inscrivent ces gènes et l'analyse des régulateurs en amont de ces voies. Un Z-score (variable centrée réduite) a été calculé par le logiciel par rapport aux gènes régulés de manière significative. Un Z-score >2 ou <-2 indique dans un intervalle de confiance de 99% que les voies biologiques induites ou réprimées ne le sont pas uniquement par chance, mais bien spécifiquement.

### 2) Résultats

### 2a) Défenses anti-oxydantes : activation de la voie Nrf2

Le tableau ci-dessous présente la différence d'expression des gènes qui sont impliqués dans la voie de Nrf2 de défense anti-oxydante suite au traitement des explants de peau avec l'extrait de Marjolaine, l'huile essentielle d'immortelle et leur association.

| **GENES** | | | **Expression par rapport au contrôle non traité ou au contrôle solvant ;** | | |
|---|---|---|---|---|---|
| **Symbole** | **Nom anglais** | **Fonction de la protéine codée par le gène** | **Marjolaine** | **HE Immortelle** | **Marjolaine et HE Immortelle** |
| ABCC1 | ATP binding cassette subfamily C member 1 | Permet le transport des formes réduites et oxydées du glutathion à travers la membrane plasmique et joue un rôle dans la protection contre les xénobiotiques | | | 2,566 |
| ACTA1 | actin, alpha 1, skeletal muscle | Protéines de structure | | 9,406 | 11,417 |
| ACTB | actin beta | | | | 2,025 |
| ATF4 | activating transcription factor 4 | Protéine interagissant avec Nrf2 (cofacteur) | | 2,316 | 2,671 |
| CDC34 | cell division cycle 34 | Appartient à la famille des enzymes de conjugaison de 1'ubiquitine impliqué dans la voie du protéasome et la réparation des protéines | | | 2,376 |
| DNAJA1 | DnaJ heat shock protein family (Hsp40) member A1 | Protéines chaperonnes protégeant contre le stress oxydatif | | | 2,029 |
| DNAJB1 | DnaJ heat shock protein family (Hsp40) member B1 | | | | 2,154 |
| DNAJB5 | DnaJ heat shock protein family (Hsp40) member B5 | | 2,591 | 6,794 | 13,824 |
| DNAJB9 | DnaJ heat shock protein family (Hsp40) member B9 | | | | 2,41 |
| DNAJC5 | DnaJ heat shock protein family (Hsp40) member C5 | | | | 2,094 |
| EIF2AK3 | eukaryotic translation initiation factor 2 alpha kinase 3 | Active une cascade signalétique conduisant à une réduction globale de la synthèse protéique | | | 2,07 |
| FOSL1 | FOS like 1, AP-1 transcription factor subunit | Les protéines FOS sont des régulateurs de la prolifération et différentiation cellulaire | | 2,707 | 3,519 |
| FTH1 | ferritin heavy chain 1 | Ces gènes codent pour des sous-unités de la ferritine, protéine majeure pour le stockage intracellulaire des ions fer | | | 2,11 |
| FTL | ferritin light chain | | | 2,601 | 4,68 |
| GSK3B | glycogen synthase kinase 3 beta | Pourrait être impliquée dans la dégradation de Nrf2 | | | 2,485 |
| GSTO1 | glutathione S-transferase omega 1 | Activité anti-oxydante | | | 2,246 |
| HERPUD 1 | homocysteine inducible ER protein with ubiquitin like domain 1 | Joue un rôle dans la dégradation des protéines | | | 2,252 |
| HMOX1 | heme oxygenase 1 | Joue un rôle essentiel dans le catabolisme de l'hème | | 2,111 | 3,87 |
| HSPB8 | heat shock protein family B (small) member 8 | Contribue à la dégradation protéolytique des protéines présentant des défauts de repliement | | 2,062 | 2,57 |
| IRS2 | insulin receptor substrate 2 | Transmet des signaux mitogéniques and anti-apoptotiques à partir du récepteur de l'insuline | | | 2 |
| MAF | MAF bZIP transcription factor | Régule négativement l'expression génique des enzymes de détoxification en se dimérisant avec MAFG. Empêche la dimérisation de MAFG et NRF2 | -2,411 | -3,486 | -5,825 |
| MAFG | MAF bZIP transcription factor G | Se dimérise avec Nrf2 facilitant son attachement aux éléments de réponse antioxydant présent dans la région promotrice des gènes régulés par Nrf2 | | 3,199 | 4,374 |
| MAP3K5 | mitogen-activated protein kinase kinase kinase 5 | Impliquée dans la réponse au stress oxydant | | | -2,106 |
| MAPK8 | mitogen-activated protein kinase 8 | Code pour la protéine JNK1. Nrf2 est transportée vers le noyau sous le contrôle des voies de signalisation ERK et JNK | | 2,048 | 3,23 |
| MAPK14 | mitogen-activated protein kinase 14 | Voie de signalisation impliquée dans la prolifération et différenciation | | 2,565 | 2,649 |
| NQ02 | N-ribosyldihydronicotinamide: quinone reductase 2 | Enzyme de détoxification induite par l'activation de Nrf2 | | 2,201 | 2,709 |
| PIK3C2B | phosphatidylinositol-4-phosphate 3-kinase catalytic subunit type 2 beta | Joue un rôle dans la prolifération, la survie et la migration cellulaire, ainsi que le transport protéique intracellulaire | | -2,318 | -2,486 |
| PIK3C2G | phosphatidylinositol-4-phosphate 3-kinase catalytic subunit type 2 gamma | | -4,325 | -6,072 | -17,725 |
| PRKCD | protein kinase C delta | Favorise la translocation de Nrf2 vers le noyau | | | 3,192 |
| PRKCH | protein kinase C eta | | | | 2,617 |
| PRKCI | protein kinase C iota | | | | 2,064 |
| RRAS2 | related RAS viral (r-ras) oncogene homolog 2 | Pourrait activer des voies de signalisation contrôlant une croissance cellulaire normale ainsi que la différenciation | | 2,67 | 3,206 |
| SQSTM1 | sequestosome 1 | Fait partie du complexe p62/SQSTM1, ce complexe est activé par Nrf2. p62 séquestre Klf1 pour une dégradation autophagique permettant ainsi la stabilisation de Nrf2 | | | 2,364 |
| TXNIP | thioredoxin interacting protein | Inhibe la fonction anti-oxydante de la thioredoxine résultant en une accumulation des espèces oxygénées oxydatives | | -3,765 | -3,875 |
| TXNRD1 | thioredoxin reductase 1 | Protège les cellules du stress oxydatif et est induit par Nrf2 | | 5,323 | 7,679 |

Le Z-score de la voie NRF-2 de défense anti-oxydante en réponse au traitement des explants de peau par l'extrait de Marjolaine, l'huile essentielle d'immortelle et leur association a été calculé par le logiciel utilisé et est indiqué dans le Tableau 2 ci-dessous.

**Tableau 2**

| | Extrait de Marjolaine | Huile Essentielle (HE) Immortelle | Extrait de Marjolaine et HE Immortelle |
|---|---|---|---|
| Voie NRF-2 | -1 | 1,069 | 2,449 |

De ce tableau, il ressort que l'extrait de marjolaine, utilisé seul, a tendance à inhiber la voie NRF-2, au contraire de l'huile essentielle d'immortelle. Ces effets ne sont toutefois pas significatifs. En revanche, il apparaît que la combinaison des deux extraits résulte en une nette activation de la voie NRF-2, ce qui démontre l'effet synergique de ces deux extraits.

### 2b) Différenciation épidermique: Augmentation de la fonction barrière

Le Tableau 3 ci-dessous présente la différence d'expression des gènes qui sont impliqués dans la différenciation épidermique suite au traitement des explants de peau avec l'extrait de Marjolaine, l'huile essentielle d'immortelle et leur association.

| **GENES** | | | **Expression par rapport au contrôle non traité ou au contrôle solvant** | | |
|---|---|---|---|---|---|
| **Symbole** | **Nom anglais** | **Fonction de la protéine codée par le gène** | **Marjolaine** | **HE Immortelle** | **Marjolaine et HE Immortelle** |
| ABCA12 | ATP binding cassette subfamily A member 12 | Impliquée dans le transport du cholestérol au niveau de la peau et est impliqué dans le maintien de la barrière épidermique lipidique | 2,018 | 2,427 | 3,398 |
| ALOXE3 | arachidonate lipoxygenase 3 | Intervient dans une voie métabolique critique pour le maintien de la fonction de barrière de la peau | | | 2,263 |
| ANXA1 | annexin A1 | Localisation de cette protéine dans les kératinocytes enrichis en tonofilaments. Joue un rôle dans la formation du cytosquelette des kératinocytes. | | 2,634 | 4,958 |
| CAMP | cathelicidin antimicrobial peptide | Joue un rôle essentiel dans la défense immunitaire de la peau contre les infections bactériennes | 4,17 | | 4,291 |
| CDH1 | cadherin 1 | Appartient à la famille des cadhérines épithéliales qui sont des protéines d'adhésion dépendantes du calcium et formant des jonctions intercellulaires. CDH1 est uniquement fonctionnellement active dans les kératinocytes différenciés formant des jonctions intercellulaires organisées. | | | 2,019 |
| CERS3 | ceramide synthase 3 | Synthétise certains céramides de la peau. Maintien de la fonction barrière. | | | 2,007 |
| CNFN | cornifelin | Constituant de l'enveloppe cornée | 4,441 | | 8,02 |
| CYP27B1 | cytochrome P450 family 27 subfamily B member 1 | Joue un rôle important dans le métabolisme de la vitamine D et le maintien de l'homéostasie calcique et la différenciation épidermique | 2,361 | 3,835 | 4,071 |
| DEFB4A/ DEFB4B | defensin beta 4A | Peptide antimicrobien qui participe à la défense immunitaire de la peau | 13,089 | | 15,134 |
| DSG3 | desmoglein 3 | Glycoproteine transmembranaire dépendante du calcium se liant aux autres protéines composant les desmosomes. Elle est principalement exprimée dans la partie basale de l'épiderme | | 2,938 | 6,183 |
| EGF | epidermal growth factor | Facteur de croissance qui stimule la prolifération et la différenciation des kératinocytes | | 2,638 | |
| ELF3 | E74 like ETS transcription factor 3 | Facteur de transcription impliqué dans la différenciation épidermique | 4,481 | 4,632 | 17,117 |
| ELOVL4 | ELOVL fatty acid elongase 4 | Participe à la synthèse des céramides épidermiques en catalysant la première réaction du cycle d'allongement des acides gras à longue chaîne. | 2,761 | 3,134 | 7,512 |
| EPHA2 | EPH receptor A2 | Récepteur à tyrosine kinase déclenchant la différenciation des kératinocytes. | 2,356 | 3,81 | 3,63 |
| EREG | epiregulin | Membre de la famille des facteurs de croissance épidermique (EGF). Facteur de croissance régulant la prolifération et la différenciation des kératinocytes. | 2,166 | 2,129 | 6,381 |
| ERRFI1 | ERBB receptor feedback inhibitor 1 | Régulateur de la différenciation épidermique, participe au maintien de la barrière épidermique | | | 2,155 |
| FABP5 | fatty acid binding protein 5 | Induit la différenciation des kératinocytes en augmentant l'activité transcriptionnelle de récepteurs activés par les peroxysomes (PPARs) en transportant les acides gras directement aux PPARs | | | 2,182 |
| FLG | filaggrin | La filaggrine (synthétisée sous la forme d'un précurseur, la profilaggrine) est une protéine dont le rôle est d'agréger les filaments intermédiaires de kératine lors de la formation de l'enveloppe cornée | -2,633 | | |
| FGF7 | fibroblast growth factor 7 | Facteur de croissance spécifique des cellules épithéliales (également nommé KGF pour "Keratinocyte Growth Factor") dont l'activité mitogénique est principalement démontrée dans les kératinocytes. | | 2,078 | |
| GRHL1 | grainyhead like transcription factor 1 | Facteur de transcription impliqué dans le développement épithélial. Régulateur de la différenciation épidermique, participe au maintien de la barrière épidermique | | | 2,651 |
| GRHL2 | grainyhead like transcription factor 1 | Inhibe la différenciation des kératinocytes par des mécanismes épigénétiques | | -2,393 | -3,865 |
| GRHL3 | grainyhead like transcription factor 3 | Protéine essentielle pour la formation la barrière épidermique | 2,149 | 2,888 | 8,2 |
| HBP1 | HMG-box transcription factor 1 | Impliquée dans la différenciation des kératinocytes | | | 2,066 |
| HGF | hepatocyte growth factor | Facteur de croissance régulant la croissance cellulaire, la motilité cellulaire et la morphogenèse. Il stimule la mitose notamment lors de la régénération tissulaire | | | 3,197 |
| HOXA7 | homeobox A7 | Réprime l'expression de gènes régulant la différenciation lors de la prolifération des kératinocytes | | | -2,423 |
| ITGB1 | integrin subunit beta 1 | Protéine d'adhésion des kératinocytes basaux à la jonction épidermique. Composant des hémidesmosomes | | | 2,067 |
| JUP | junction plakoglobin | Composante cytoplasmique majeure des desmosomes | | | 2,204 |
| KLF5 | Kruppel like factor 5 | Facteur de transcription augmentant la prolifération des kératinocytes | | | 2,298 |
| KRT6B | Keratin 6B | Protéine de structure impliquée dans la kératinisation de l'épiderme | | | 2,455 |
| LATS2 | large tumor suppressor kinase 2 | Interagit avec les protéines des centrosomes, elle est essentielle l'initiation de la mitose et impliquée dans la différenciation des kératinocytes | | | 2,411 |
| LCE3A | late cornified envelope 3A | Composant protéique de l'enveloppe cornée | 9,888 | | 11,481 |
| LCE3C | late cornified envelope 3C | | 6,177 | | 8,285 |
| LCE3D | late cornified envelope 3D | | 4,761 | 2,136 | 8,197 |
| LCE3E | late cornified envelope 3E | | 5,068 | | 5,917 |
| LOR | loricrin | Composant protéique majeur de l'enveloppe cornée | -2,097 | | |
| OVOL2 | ovo like zinc finger 2 | Régule la prolifération cellulaire dans la couche basale de l'épiderme | -2,291 | | -2,853 |
| PLK1 | polo like kinase 1 | Fortement exprimée durant la mitose, elle est nécessaire à la prolifération cellulaire | | -3,252 | -2,383 |
| PPARA | peroxisome proliferator activated receptor alpha | Régule la différenciation épidermique | | 2,077 | 2,779 |
| PPARD | peroxisome proliferator activated receptor delta | | 2,764 | 4,155 | 8,966 |
| PRKCH | protein kinase C eta | Régule la différenciation des kératinocytes | | | 2,617 |
| S100A7 | S100 calcium binding protein A7 | Composants protéiques de l'enveloppe cornée | 2,717 | | 3,22 |
| S100A11 | S100 calcium binding protein A11 | | | | 2,019 |
| SPRR1A | small proline rich protein 1A | Composants de l'enveloppe cornée | 4,665 | 5,288 | 16,546 |
| SPRR1B | small proline rich protein 1B | | 2,544 | 3,095 | 4,893 |
| TGM1 | transglutaminase 1 | Impliquées dans la formation de l'enveloppe cornée en créant des pontages entre les protéines qui la constituent. | 4,557 | 3,819 | 8,105 |
| TGM3 | transglutaminase 3 | | | | 6,104 |
| TP63 | tumor protein p63 | Facteur de transcription impliqué dans le développement de la peau qui inhibe la différentiation épidermique | | -3,148 | -7,121 |
| WNT16 | Wnt family member 16 | L'expression de WNT16B augmente le taux de cellules en prolifération et prolonge la clonogénicité des kératinocytes primaires | -2,096 | -10,838 | -17,812 |
| ZFP36 | ZFP36 ring finger protein | Influence la différenciation des kératinocytes | | | 2,527 |

Ce tableau montre que certains gènes impliqués dans la différenciation des kératinocytes sont régulés positivement et d'autres inhibant la différenciation des kératinocytes sont régulés négativement. De plus, des gènes des facteurs de croissance (HGF, épiréguline) sont également régulés positivement permettant un maintien de la réserve des kératinocytes proliférateurs. L'ensemble de ces gènes va permettre un renforcement de la barrière épidermique.

### 3) Conclusion

Cet exemple démontre par une analyse transcriptomique *ex-vivo* par puces à ADN que la composition de l'invention permet de stimuler les gènes impliqués notamment dans le renouvellement de l'épiderme et la gestion du stress oxydatif, permettant ainsi de lutter contre le vieillissement de la peau dû en particulier aux agressions extérieures générant un stress oxydatif, et à renforcer la fonction barrière de la peau.

### Exemple 2 : Composition cosmétique

La composition suivante est préparée de manière classique pour l'homme de l'art, en mélangeant les ingrédients ci-dessous dans les proportions pondérales indiquées.

| | |
|---|---|
| Huile essentielle d'immortelle | 0,01-0,1 % |
| Extrait de feuilles de marjolaine | 0,2-0,8 % |
| Adénosine | 0,05 % |
| Huiles | 5-15 % |
| Gélifiants huileux | 1-5 % |
| Gélifiants aqueux | 1-3 % |
| Polyols | 20-25 % |
| Séquestrant | qs |
| Anti-oxydants | qs |
| Parfum | qs |
| Conservateurs | qs |
| Eau | qsp 100 % |

## Revendications

1. Composition cosmétique comprenant une huile essentielle d'immortelle et un extrait terpénique d'au moins une plante du genre *Origanum.*

2. Composition selon la revendication 1, **caractérisée en ce que** l'immortelle est choisie parmi les espèces *Helichrysum italicum, Helichrysum arenarium* et *Helichrysum stoechas,* de préférence *Helichrysum italicum.*

3. Composition selon la revendication 1 ou 2, **caractérisée en ce que** l'huile essentielle d'immortelle est obtenue à partir des parties aériennes de la plante, en particulier de ses fleurs ou sommités fleuries.

4. Composition selon l'une quelconque des revendications 1 à 3, **caractérisée en ce que** la plante du genre *Origanum* appartient à l'espèce *Origanum majorana.*

5. Composition selon l'une quelconque des revendications 1 à 4, **caractérisée en ce que** l'extrait *d'Origanum* est obtenu à partir des parties aériennes de la plante choisies parmi la tige, les feuilles et leurs mélanges, plus préférentiellement les feuilles.

6. Composition selon l'une quelconque des revendications 1 à 5, **caractérisée en ce que** l'extrait *d'Origanum* est obtenu par extraction à l'aide d'un solvant polaire protique comprenant, de préférence constitué par, au moins un solvant choisi parmi l'eau, un mono-alcool tel que l'éthanol ou l'isopropanol, un glycol tel que le propylène glycol ou le butylène glycol, un polyol tel que le xylitol et leurs mélanges.

7. Utilisation de la composition selon l'une quelconque des revendications 1 à 6 pour lutter contre les signes du vieillissement cutané, notamment la formation de rides, le relâchement cutané et la perte d'éclat du teint, dus en particulier à l'exposition aux UV et/ou à la lumière bleue, au tabac et/ou à la pollution et/ou pour lutter contre le dessèchement de la peau.

8. Procédé cosmétique pour lutter contre les signes du vieillissement cutané, notamment la formation de rides, le relâchement cutané et la perte d'éclat du teint, dus en particulier à l'exposition aux UV et/ou à la lumière bleue, au tabac et/ou à la pollution, et/ou pour lutter contre le dessèchement de la peau, comprenant l'application topique sur la peau de la composition selon l'une quelconque des revendications 1 à 6.

## Patentansprüche

1. Kosmetische Zusammensetzung, die ein ätherisches Immortelleöl und einen Terpenextrakt mindestens einer Pflanze der Gattung *Origanum* umfasst.

2. Zusammensetzung nach Anspruch 1, **dadurch gekennzeichnet, dass** die Immortelle aus den Arten *Helichrysum italicum, Helichrysum arenarium* und *Helichrysum stoechas,* vorzugsweise *Helichrysum italicum,* ausgewählt ist.

3. Zusammensetzung nach Anspruch 1 oder 2, **dadurch gekennzeichnet, dass** das ätherische Immortelleöl aus den oberirdischen Teilen der Pflanze, insbesondere ihren Blüten oder Stängelspitzen mit Blüten, erhalten wird.

4. Zusammensetzung nach einem der Ansprüche 1 bis 3, **dadurch gekennzeichnet, dass** die Pflanze der Gattung *Origanum* zur Art *Origanum majorana* gehört.

5. Zusammensetzung nach einem der Ansprüche 1 bis 4, **dadurch gekennzeichnet, dass** der *Origanum-Extrakt* aus den oberirdischen Teilen der Pflanze erhalten wird, die aus dem Stängel, den Blättern und Mischungen davon, weiter bevorzugt den Blättern, ausgewählt sind.

6. Zusammensetzung nach einem der Ansprüche 1 bis 5, **dadurch gekennzeichnet, dass** der *Origanum-Extrakt* durch Extraktion mit einem protischen polaren Lösungsmittel erhalten wird, das mindestens ein Lösungsmittel umfasst, vorzugsweise daraus besteht, das aus Wasser, einem einwertigen Alkohol wie Ethanol oder Isopropanol, einem Glycol wie Propylenglycol oder Butylenglycol, einem Polyol wie Xylit und Mischungen davon ausgewählt ist.

7. Verwendung der Zusammensetzung nach einem der Ansprüche 1 bis 6 zur Bekämpfung der Symptome der Hautalterung, insbesondere der Bildung von Falten, der Hauterschlaffung und des Verlusts des strahlenden Teints, insbesondere aufgrund der Exposition gegenüber UV und/oder blauem Licht, Tabak und/oder der Umweltverschmutzung, und/oder zur Bekämpfung der Austrocknung der Haut.

8. Kosmetisches Verfahren zur Bekämpfung der Symptome der Hautalterung, insbesondere der Bildung von Falten, der Hauterschlaffung und des Verlusts des strahlenden Teints, insbesondere aufgrund der Exposition gegenüber UV und/oder blauem Licht, Tabak und/oder der Umweltverschmutzung, und/oder zur Bekämpfung der Austrocknung der Haut, das die topische Anwendung der Zusammensetzung nach einem der Ansprüche 1 bis 6 auf der Haut umfasst.

## Claims

1. A cosmetic composition comprising an essential oil of Immortelle and a terpene extract of at least one plant of the *Origanum* genus.

2. The composition as claimed in claim 1, **characterized in that** the Immortelle is chosen from the species *Helichrysum italicum, Helichrysum arenarium* and *Helichrysum stoechas,* preferably *Helichrysum italicum.*

3. The composition as claimed in claim 1 or 2, **characterized in that** the essential oil of Immortelle is obtained from the aerial parts of the plant, in particular from its flowers or flowering heads.

4. The composition as claimed in any one of claims 1 to 3, **characterized in that** the plant of the *Origanum* genus belongs to the species *Origanum majorana.*

5. The composition as claimed in any one of claims 1 to 4, **characterized in that** the extract of *Origanum* is obtained from the aerial parts of the plant chosen from the stem, the leaves and mixtures thereof, more preferentially the leaves.

6. The composition as claimed in any one of claims 1 to 5, **characterized in that** the extract of *Origanum* is obtained by extraction using a protic polar solvent comprising, preferably consisting of, at least one solvent chosen from water, a monoalcohol such as ethanol or isopropanol, a glycol such as propylene glycol or butylene glycol, a polyol such as xylitol and mixtures thereof.

7. The use of the composition as claimed in any one of claims 1 to 6, for combating the signs of skin aging, in particular the formation of wrinkles, the slackening of the skin and the loss of radiance of the complexion, caused in particular by exposure to UV radiation and/or to blue light, to smoking and/or to pollution and/or for combating dryness of the skin.

8. A cosmetic process for combating the signs of skin aging, in particular the formation of wrinkles, the slackening of the skin and the loss of radiance of the complexion, caused in particular by exposure to UV radiation and/or to blue light, to smoking and/or to pollution, and/or for combating dryness of the skin, comprising the topical application of the composition as claimed in any one of claims 1 to 6 to the skin.
